(19) Europäisches Patentamt / European Patent Office / Office européen des brevets

(11) **EP 2 704 620 B1**

(12) **FASCICULE DE BREVET EUROPEEN**

(45) Date de publication et mention
de la délivrance du brevet:
**28.06.2017 Bulletin 2017/26**

(21) Numéro de dépôt: **12713211.6**

(22) Date de dépôt: **08.03.2012**

(51) Int Cl.:
*A61B 3/10* (2006.01)     *A61B 5/103* (2006.01)
*A61B 3/113* (2006.01)

(86) Numéro de dépôt international:
**PCT/FR2012/050482**

(87) Numéro de publication internationale:
**WO 2012/153022 (15.11.2012 Gazette 2012/46)**

(54) **PROCEDE DE DETERMINATION DE LA DISTANCE DE LECTURE**

VERFAHREN ZUR BESTIMMUNG EINER LESEENTFERNUNG

METHOD FOR DETERMINING READING DISTANCE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **06.05.2011 FR 1153881**

(43) Date de publication de la demande:
**12.03.2014 Bulletin 2014/11**

(73) Titulaire: **Essilor International
(Compagnie Générale d'Optique)
94220 Charenton-le-Pont (FR)**

(72) Inventeurs:
• **HADDADI, Ahmed
F-94220 Charenton le Pont (FR)**

• **DELZERS, Jean
F-94220 Charenton le Pont (FR)**

(74) Mandataire: **Allain, Laurent et al
Ipsilon
Le Centralis
63, avenue du Général Leclerc
92340 Bourg-la-Reine (FR)**

(56) Documents cités:
FR-A1- 2 931 002     US-A1- 2007 229 761
US-A1- 2008 294 017     US-A1- 2011 090 712
US-B1- 7 909 460

EP 2 704 620 B1

## Description

L'invention se rapporte à un procédé de détermination de la distance de lecture pour un individu amené à lire un texte standard. La notion de texte standard est large car le procédé selon l'invention doit pouvoir notamment s'adresser à des individus ne sachant pas lire. Par conséquent, un texte standard peut inclure un texte écrit usuel, ou un motif simplifié ou une figure pouvant être couramment rencontrés dans un livre ou une revue. Il est important, notamment pour un opticien, de connaître la distance de lecture, pour incorporer ce paramètre dans la réalisation d'une paire de lunettes.

Pour une meilleure compréhension de la description, les expressions « procédé de détermination de la distance de lecture » et « test de détermination de la distance de lecture » sont équivalentes.

Les procédés de détermination de la distance de lecture reposent généralement sur une évaluation sommaire et approximative de celle-ci, réalisée au jugé sans outillage spécifique et donc sans rigueur particulière. La plupart du temps, ces procédés ont pour unique objectif une détermination rapide de cette distance, sans tenir compte de la posture adoptée par l'individu. Or, chaque individu, en fonction de sa taille et de sa morphologie, va être amené à incliner son corps et/ou sa tête de façon plus ou moins marquée, et à orienter au plus juste le support de lecture dans l'espace, pour se retrouver dans des conditions de lecture optimales, lui octroyant le maximum de confort. Pour être rigoureuse, la mesure de la distance de lecture doit intégrer tous ces ajustements de position et d'orientation du corps et du support de lecture. Le terme « support de lecture » est général et désigne tout dispositif sur lequel est apposé un texte ou des figures, et peut inclure par exemple, un livre ou une revue.

Le document FR 2 931 002 A1 décrit un ensemble et un procédé de mesure de l'écart pupillaire et de la distance de lecture. Les procédés de détermination de la distance de lecture selon l'invention, sont complets et précis, car, d'une part, ils tiennent compte de la posture adoptée par l'individu ainsi que de l'inclinaison du support de lecture, et d'autre part, ils permettent de restituer automatiquement l'information concernant la distance de lecture. Ils sont de plus conviviaux et rapides, en proposant au lecteur d'adopter une position de lecture confortable, sans contrainte particulière et sans équipement spécifique, la distance de lecture étant communiquée à l'individu dans la continuité du procédé, juste après la phase de lecture.

Plus spécifiquement, l'invention a pour objet un procédé de détermination de la distance de lecture pour un individu amené à observer une zone de lecture, tel que défini dans la revendication 1. Le principe d'un procédé selon l'invention, consiste à capturer au moins une image de l'individu en train de lire une zone de lecture sur la tablette, puis à repérer la position de chaque marqueur de la tablette et celle du repère facial sur ladite au moins une image. Connaissant la distance de l'individu par rapport à l'appareillage, et à partir de considérations angulaires induites par les repérages précédents, il devient possible de déterminer précisément la distance de lecture. Il est supposé que la distance séparant le repère facial de l'individu au plan de ses yeux, ainsi que la distance séparant le marqueur et la zone de lecture sont connues avec précision, afin de les intégrer dans le calcul de la distance de lecture. La tablette de lecture est un objet léger, pouvant être facilement manipulé, et sur lequel est apposée la zone de lecture. Dans le cas où l'individu ne saurait pas lire, la zone de lecture peut être matérialisée par un dessin ou une figure simplifiée pouvant avantageusement remplacer un texte écrit. Chaque marqueur de la tablette de lecture est placé au recto de celle-ci, de manière à apparaître sur un film ou une photo lorsque l'individu incline ladite tablette pour déchiffrer le texte et/ou les dessins. L'appareillage peut être fixe ou mobile. La restitution de la distance de lecture peut s'effectuer par tous les moyens possibles, qu'ils soient oraux ou visuels, l'essentiel étant que l'information soit claire et qu'elle soit effectuée dans la continuité du traitement d'images, sans période d'attente. Le repère facial peut, soit être naturel en étant représenté par une partie du visage facilement repérable sur une image, comme par exemple la pupille des yeux, soit être artificiel par l'intermédiaire d'au moins un marqueur. En ce qui concerne la phase de lecture en elle-même, aucune contrainte de posture ou de placement n'est imposée à l'individu, celui-ci disposant d'une liberté totale pour se retrouver dans des conditions optimales de confort et de lecture, la seule limite étant qu'il demeure en face de l'appareillage. De cette manière, l'individu trouvera un compromis acceptable entre une inclinaison de la tête et/ou du buste, et un positionnement spatial adapté de la tablette.

Préférentiellement, l'individu porte une paire de lunettes, et le repère facial est matérialisé par des marqueurs solidarisés à un clip fixé sur ladite paire de lunettes. De cette manière, les lunettes servent de support aux marqueurs, dont la proximité avec les yeux leur permettra d'être représentatifs de ceux-ci sur une image. Le clip peut porter, soit un marqueur individuel positionné entre les deux yeux, soit deux marqueurs placés au droit de chaque oeil, le nombre de marqueurs n'étant pas limité. Généralement, ces marqueurs sont légèrement décalés par rapport à la position des yeux, ce décalage étant de faible amplitude et bien connu pour pouvoir être intégré dans le calcul de la distance de lecture. La tablette est dotée de trois marqueurs de repérage, l'un étant en position frontale sur ladite tablette, les deux autres étant en position latérale. Ces marqueurs permettent de repérer la position de la tablette sur une image, en particulier lorsque celle-ci est très inclinée au moment de la phase de lecture par l'individu. Autrement dit, si la tablette pouvait se ramener à une pièce de faible épaisseur présentant un recto et un verso, la zone de lecture serait située sur le verso, et les marqueurs seraient apposés sur le recto.

De façon préférentielle, le moyen mis en oeuvre pour acquérir au moins une image est une caméra. En effet, une caméra est le moyen de capture d'images le plus complet et le plus souple, permettant de produire un film en continu, ou d'effectuer divers arrêts sur images permettant de sélectionner les meilleures images. Ce type d'appareil permet également d'enchaîner en continu les prises de vue, en mode zoomé ou à distance.

Selon un premier mode de réalisation préféré d'un procédé selon l'invention, le repère facial de l'individu ainsi que chaque repère de la tablette, apparaissent simultanément sur une même image. Pour cette configuration, il est supposé que la résolution est suffisamment bonne pour pouvoir distinguer avec précision à la fois le repère facial de l'individu et chaque marqueur de la tablette de lecture, et dans cette hypothèse, une seule prise de vue suffit pour déterminer la distance de lecture.

Selon un autre mode de réalisation préféré d'un procédé selon l'invention, la caméra acquiert au moins deux images, une première image sur laquelle apparait au moins le repère facial de l'individu, et une deuxième image sur laquelle apparait chaque marqueur de la tablette, le traitement par calcul s'effectuant à partir des informations recueillies sur lesdites images. Pour cette configuration, il peut être supposé que la résolution n'est pas suffisamment bonne de loin, au point de ne pas pouvoir distinguer sur un même cliché le repère facial de l'individu et chaque marqueur de la tablette.

Avantageusement, la caméra est déplacée entre une première position destinée à la capture de la première image et une deuxième position destinée à la capture de la deuxième image. En effet, il peut tout simplement arriver que la caméra ne soit pas en mesure de prendre un cliché suffisamment bien défini, et sur lequel apparaissent à la fois le repère facial de l'individu et chaque marqueur de la tablette de lecture. Dans ces conditions, la caméra acquiert une première vue de l'un des deux éléments, le repère facial ou chaque marqueur de la tablette, puis est ensuite déplacée pour venir acquérir une deuxième vue de l'autre élément, le traitement de ces deux images intégrant les deux positions de la caméra dans l'espace. En effet, afin de pouvoir associer efficacement les informations recueillies sur les deux clichés, en vue de déterminer la distance de lecture, il est impératif de connaitre les coordonnées de la caméra dans l'espace, au moment de la prise de ces deux clichés.

De façon avantageuse, le traitement de ladite au moins une image, comprend une étape de soustraction entre un premier vecteur reliant le repère facial de l'individu et l'appareil d'acquisition de l'image, et un deuxième vecteur reliant la zone de lecture sur la tablette et ledit appareil, la restitution de l'information s'effectuant au moyen d'une lecture directe sur un écran. Ladite étape de soustraction des vecteurs est complétée par des ajustements angulaires et des ajustements de vecteurs pour tenir compte des différentes inclinaisons introduites lors de la phase de lecture, et des décalages entre le repère facial et le plan des yeux, et entre chaque marqueur de la tablette et la zone de lecture.

L'invention se rapporte également à une tablette de lecture portable pour la mise en oeuvre d'un procédé selon l'invention, ladite tablette comprenant une face destinée à la lecture, et au moins un marqueur de repérage spatial de ladite tablette, ledit au moins un marqueur étant configuré pour permettre un repérage même par faible luminosité. La principale caractéristique d'une tablette selon l'invention, est qu'elle comprend une première face de lecture prolongée par une deuxième face inclinée d'un angle de 10° à 80° par rapport à ladite première face, ladite deuxième face comprenant trois marqueurs, l'un en position frontale, et les deux autres étant en position latérale, lesdits trois marqueurs permettant de repérer la position dans l'espace de ladite tablette. La face de lecture peut indifféremment comporter un texte, des lettres séparées ou des chiffres, ou des figures. De façon générale, cette face comporte tout type de caractères pouvant être lus par un individu. Le marqueur est préférentiellement placé au dos de cette tablette par rapport à la face de lecture, pour pouvoir repérer ladite tablette sur une photo, même lorsqu'elle est très inclinée au moment de la lecture. La géométrie du marqueur associée à sa teinte doit lui permettre d'être repérer facilement et nettement, avec toute sorte de luminosité, y compris les plus faibles. La tablette comprend une première face de lecture prolongée par une deuxième face inclinée d'un angle de 10° à 80° par rapport à ladite première face, ladite deuxième face comprenant au dos trois marqueurs, l'un en position frontale, et les deux autres étant en position latérale, lesdits trois marqueurs permettant de repérer spatialement ladite tablette. Le terme «face» désigne ici une paroi. De cette manière, lorsque la tablette est inclinée au moment de la lecture, les marqueurs demeureront bien visibles sur une image ou un film pris de face.

Les procédés de détermination de la distance de lecture selon l'invention présentent l'avantage d'être complets, en proposant, outre la mise en oeuvre d'un test simple, rapide et peu contraignant, une ultime étape constituant à restituer automatiquement et de façon précise à l'individu le résultat du test. Ils présentent de plus l'avantage d'être conviviaux, en mettant en scène un individu qui va être filmé ou pris en photo, avec des lunettes équipées d'un clip de marquage. Enfin, ces procédés sont sûrs, fiables et parfaitement répétitifs.

On donne ci-après, une description détaillée d'un mode de réalisation préféré d'un procédé de détermination de la distance de lecture selon l'invention, ainsi que d'une tablette de lecture selon l'invention, en se référant aux figures 1 à 3c.

- La figure 1 est une vue latérale d'une installation prévue pour appliquer le procédé selon l'invention, et montrant un individu en phase de lecture,

- La figure 2 est une vue latérale d'une tablette de lecture selon l'invention, et montrant les différents repères et angles reliant la zone de lecture aux marqueurs de la tablette,

- La figure 3a est une vue de face d'une tablette de lecture selon l'invention,

- La figure 3b est une vue du dessus de la tablette de la figure 3a,

- La figure 3c est une vue de profil de la tablette de la figure 3a,

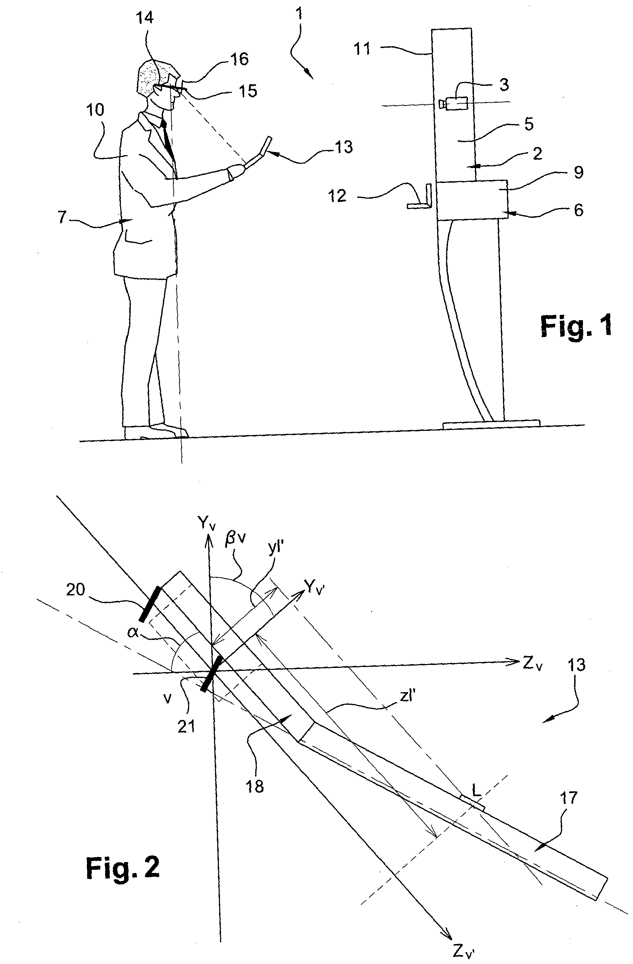

En se référant à la figure 1, une installation 1 destinée à mettre en oeuvre un procédé de détermination de la distance de lecture selon l'invention, fait intervenir un appareillage 2 doté d'une caméra 3 permettant d'acquérir au moins une image, de moyens 5 de traitement de ladite image et de moyens 6 de restitution de ladite image traitée. L'appareillage 2 se présente sous la forme d'une colonne verticale et fixe, comprenant la caméra 3, dont le positionnement en hauteur est ajustable en fonction de la taille de l'individu 7 se soumettant au test. Idéalement la hauteur de la caméra 3 est réglée de façon à être alignée horizontalement avec les yeux de l'individu 2. Les moyens 6 de restitution de l'image traitée, comportent un écran 9 de visualisation, qui peut, soit être directement incorporé dans la colonne 2, soit être relié à celle-ci par les cordons appropriés. Avantageusement l'écran 9 est orienté vers l'individu 7, afin qu'il puisse directement visualiser sa propre image, et lire, sans bouger, la distance de lecture calculée par l'appareillage 2, cette information apparaissant explicitement et instantanément sur ledit écran 9. L'individu 7 peut se soumettre au test de détermination de sa distance de lecture selon l'invention, en étant en position debout ou en position assise, la distance séparant son buste 10 de la caméra 3 étant approximativement de 1,3m. La colonne 2 dispose d'un miroir 11 sans tain et plan, camouflant la caméra 3 et permettant à l'individu 7 de se positionner correctement devant ladite colonne 2, en se référant à sa propre image renvoyée par ledit miroir 11, pour effectuer le test dans de bonnes conditions. Par ce biais, l'individu 7 s'évertue à rester globalement en face de la colonne 2, sans en dévier de façon significative. La colonne 2 comprend également un support 12 en saillie, permettant de poser une tablette de lecture 13 prévue pour la détermination de la distance de lecture, ce support 12 étant utilisé entre deux tests. Dans le cadre d'une prise de mesure chez l'opticien, l'individu 7 porte une monture 14 de verres correcteurs. Puisque le principe du procédé repose au moins sur l'acquisition d'une image traitée à partir de l'identification précise de la position du plan des yeux de l'individu 7, il est donc important de pouvoir repérer de façon rigoureuse, la position d'un point ou d'une zone, dont on connait parfaitement le positionnement par rapport à audit plan. Un repère facial naturel n'étant pas toujours possible en raison par exemple d'une faible luminosité, les lunettes 14 sont équipées de marqueurs 15, permettant indifféremment de repérer, soit chacun des yeux 8, soit un point central entre lesdits yeux 8. Ces marqueurs 15 sont supportés par une tige 16 pouvant venir se clipper sur la monture desdites lunettes 14, dans une position sensiblement horizontale, au dessus de chaque verre, ce clip 16 pouvant, par exemple, être analogue à celui décrit dans la demande de brevet US2009/0262302. L'appareillage peut être doté d'un moyen d'éclairage permettant d'accroître la luminosité ambiante et d'améliorer les conditions d'acquisition des images. La phase de lecture durant le procédé selon l'invention, est effectuée au moyen d'une tablette 13 de lecture portable, qui est un objet léger et de faible dimensions, que l'individu 7 tient dans sa main, et place à une certaine distance de ses yeux 8 afin d'opérer cette phase. Cette tablette 13 comporte un texte ou des dessins, dont la taille est analogue celle que l'on pourrait rencontrer dans un livre.

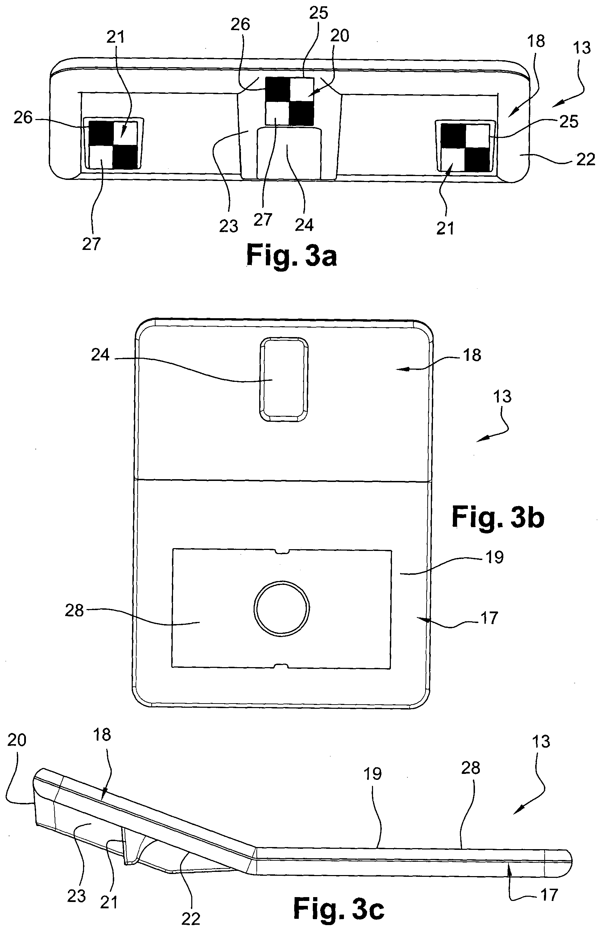

En se référant aux figures 3a,3b et 3c, une tablette de lecture 13 permettant à un individu 7 de lire un texte durant le test de détermination de la distance de lecture, est un objet ayant une épaisseur faible et constante, et qui est constitué de deux parois planes et rectangulaires 17,18, faisant entre elles un angle compris entre 10° et 80°. La première paroi 17 comprend une face 19 de lecture sur laquelle est située la zone de lecture 28, matérialisée par un texte ou des figures ou des dessins. La deuxième paroi 18, qui est inclinée par rapport à la première 17, est équipée de trois marqueurs 20,21, l'un 20 étant en position centrale et frontale et les deux autres 21 étant en position latérale. De façon plus précise, une face 22 de la deuxième paroi 18 est dotée d'une protubérance 23 annulaire et centrale, délimitant une ouverture 24 traversant ladite deuxième paroi 18, le marqueur 20 en position centrale étant située sur ladite protubérance 23. Si l'on suppose que la deuxième paroi 18 est placée à l'avant de la première paroi 17, le marqueur central 20 se retrouve dans la partie la plus avancée de la deuxième paroi 18. Les trois marqueurs 20,21 sont identiques est sont constitués chacun d'un carré 25 subdivisé en quatre carrés 26,27 de plus petite dimension, deux petits carrés 26 situés sur la même diagonale étant teints en foncé, les deux autres 27 situés sur l'autre diagonale étant teints en clair. De façon avantageuse, les carrés 26,27 se distinguent entre eux par un dégradé de gris. De tels marqueurs 20,21 peuvent être facilement repérés, quelle que soit la luminosité ambiante. Ces trois marqueurs 20,21 sont inclinés par rapport au plan de la deuxième paroi 18 et sont placés sur la face 22 de ladite deuxième paroi 18 qui est opposée à la face 19 de la première paroi 17 comportant la zone de lecture 28. L'inclinaison entre les deux parois 20,21 ainsi que le positionnement des marqueurs 20,21, permettent à la tablette 13 d'être repérée sur un film ou une photo prise par la caméra 3, lors de la phase de lecture par l'individu 7, et au cours de laquelle ladite tablette 13 risque de se retrouver inclinée.

En se référant à la figure 2, le traitement par calcul de l'image, pour déterminer la distance de lecture, consiste d'abord à déterminer le vecteur reliant les marqueurs 15 du clip 16 à la caméra 3, puis à déterminer le vecteur reliant les marqueurs 20,21 de la tablette 13 à ladite caméra 3. Ces deux vecteurs sont ensuite retranchés l'un de l'autre pour obtenir un troisième vecteur dont la longueur est la distance séparant les deux familles 15,20,21 de marqueurs. Enfin, cette distance est complétée par les distances séparant les marqueurs 15 du clip 16 au plan des yeux, et par la distance séparant les marqueurs 20,21 de la tablette 13 à la zone de lecture 28. La distance séparant les marqueurs 15 du clip 16 au plan des yeux est généralement faible, de un à quelques centimètres, et peut facilement être connue avec précision. En revanche, le vecteur reliant les marqueurs 20,21 de la tablette 13 avec la zone de lecture 28 nécessite un calcul un peu plus complexe basé sur des considérations angulaires et des distances corrigées, en raison de l'inclinaison de ladite tablette 13. Le principe de la méthode de calcul de la distance de lecture est présenté ci-après :

Les points remarquables à prendre en considération, sont les suivants :

- O : le centre optique de la caméra 3. C'est l'origine du repère dans lequel toutes les mesures sont finalement rapportées ;

- V : le centre géométrique des marqueurs 21 latéraux (droit et gauche) de la tablette 13.

$(xv, yv, zv)$ : coordonnées du point V par rapport à la caméra 3, c'est-à-dire par rapport au point O
$(xv', yv', zv')$ repère lié à la tablette 13 et d'origine le point V
$(\alpha v, \beta v, \gamma v)$ : posture de lacet, tangage et roulis de la tablette 13 par rapport à la caméra 3, c'est-à-dire : angles respectifs du repère lié à la tablette 13 par rapport au repère lié à la caméra 3, pour passer de $(xv, yv, zv)$ à $(xv', yv', zv')$

- L : le centre de la zone de lecture 28
$(xl', yl', zl')$ : coordonnées du point L dans le repère lié à la tablette 13 et d'origine le point V

- C : le centre du clip 16

$(xc, yc, zc)$ : coordonnées du point C dans le repère lié à la caméra 3 d'origine le point O
$(\alpha c, \beta c, \gamma c)$ : angles d'orientation du clip 16 dans le repère lié à la caméra 3 d'origine le point O

- R : centre du segment reliant les centres de rotations des yeux

$(xr', yr', zr')$ : coordonnées du point R dans le repère lié au clip 16 et d'origine le point C, le centre du clip 16

Le repère $(Xv, Yv, Zv)$ se confond avec le repère $(Xv', Yv', Zv')$ après application de la rotation dont la matrice est le produit $Mv = Rz\,(\gamma v)\; Rx\,(\beta v)\; Ry\,(\alpha v)$ avec

$$R_{\mathbf{x}}(\theta) = \begin{bmatrix} 1 & 0 & 0 \\ 0 & \cos\theta & -\sin\theta \\ 0 & \sin\theta & \cos\theta \end{bmatrix}$$

$$R_{\mathbf{y}}(\theta) = \begin{bmatrix} \cos\theta & 0 & \sin\theta \\ 0 & 1 & 0 \\ -\sin\theta & 0 & \cos\theta \end{bmatrix}$$

$$R_{\mathbf{z}}(\theta) = \begin{bmatrix} \cos\theta & -\sin\theta & 0 \\ \sin\theta & \cos\theta & 0 \\ 0 & 0 & 1 \end{bmatrix}$$

Les coordonnées du point L étant $(xl', yl', zl')$ dans le repère $(Xv', Yv', Zv')$, le calcul des coordonnées $(xl, yl, zl)$ du point L dans le repère $(Xv, Yv, Zv)$ s'effectue en appliquant la matrice de rotation inverse de Mv :

$$Mv^{-1} = Ry\,(-\alpha v)\; Rx\,(-\beta v)\; Rz\,(-\gamma v).$$

En décomposant le vecteur $\overrightarrow{OL}$ en $\overrightarrow{OV} + \overrightarrow{VL}$, les coordonnées du point L dans le repère de la caméra sont $(xv+xl, yv+yl, zv+zl)$.

Le calcul de la position du centre du segment reliant les centres de rotation des yeux 8 dans le repère de la caméra 3 suit les étapes suivantes :

En appliquant la méthode utilisée précédemment, le repère $(Xc, Yc, Zc)$ se confond avec le repère $(Xc', Yc', Zc')$ après application de la rotation dont la matrice est le produit $Mc = Rz\,(\gamma c)\; Rx\,(\beta c)\; Ry\,(\alpha c)$.

Les coordonnées du point R étant $(xr', yr', zr')$ dans le repère $(Xc', Yc', Zc')$, le calcul des coordonnées $(xr, yr, zr)$ du point R dans le repère $(Xc, Yc, Zc)$ s'effectue en appliquant la matrice de rotation inverse de Mc :

$$Mc^{-1} = Ry\,(-\alpha c)\; Rx\,(-\beta c)\; Rz\,(-\gamma c).$$

En décomposant le vecteur $\overrightarrow{OR}$ en $\overrightarrow{OC} + \overrightarrow{CR}$, les coordonnées du point R dans le repère de la caméra sont $(xc+xr, yc+yr, zc+zr)$.

Le procédé de détermination de la distance de lecture selon l'invention, suit les étapes suivantes :

- Positionnement de l'individu 7 devant la colonne 2, en se référant à sa propre image renvoyée par le miroir 11,

- Lecture par l'individu 7 de la zone de lecture 28 en adoptant une posture naturelle et confortable, et en ajustant la position de la tablette 13,

- Acquisition par la caméra 3 d'une première image sur laquelle figure le clip 16 doté de ses marqueurs 15, puis déplacement de ladite caméra 3 pour acquérir une deuxième image sur laquelle figurent les marqueurs 20,21 de la tablette 13, chaque image étant suffisamment définie pour pouvoir distinguer de façon précise la position des différents marqueurs 15,20,21.

- Traitement par calculs des deux images,

- L'écran 9, qui est tourné vers l'individu, affiche alors la distance de lecture calculée, quelques secondes après l'acquisition des deux images.

L'invention ne se limite pas aux différents modes de réalisation décrits ci-avant, et donnés à titre d'exemples non limitatifs.


**Revendications**

1. Procédé de détermination de la distance de lecture pour un individu (7) amené à observer une zone de lecture (28), comprenant les étapes suivantes :

   - Positionnement de l'individu (7) devant un appareillage (2) conçu pour acquérir au moins une image de face dudit individu (7), puis pour traiter ladite image et enfin restituer une information permettant à l'individu (7) de connaitre sa distance de lecture,
   - Lecture par l'individu (7) de ladite zone (28) placée sur une tablette portable (13) et dotée de trois marqueurs (20,21) de repérage, l'un (20) étant en position frontale sur ladite tablette (13), les deux autres (21) étant en position latérale, en adoptant une posture naturelle et en ajustant la position de ladite tablette (13),
   - Acquisition par l'appareillage (2) d'au moins une image permettant de visualiser au moins un repère facial (15) de l'individu (7) et chaque marqueur (20,21) de la tablette (13) de lecture,
   - Traitement par calculs de ladite au moins une image, en tenant compte de la position du repère facial (15) de l'individu (7) et de la position des trois marqueurs (20,21) de ladite tablette (13),
   - Restitution par l'appareillage (2) de l'information indiquant la distance de lecture.

2. Procédé selon la revendication 1, **caractérisé en ce que** l'individu (7) porte une paire de lunettes (14), et **en ce que** le repère facial est matérialisé par des marqueurs (15), permettant indifféremment de repérer, soit chacun des yeux (8), soit un point central entre lesdits yeux (8) et solidarisés à un clip (16) fixé sur ladite paire de lunettes (14).

3. Procédé selon l'une quelconque des revendications précédente, **caractérisé en ce que** le moyen mis en oeuvre pour acquérir au moins une image est une caméra (3).

4. Procédé selon la revendication 3, **caractérisé en ce que** le repère facial (15) de l'individu (7) ainsi que chaque marqueur (20,21) de la tablette (13), apparaissent simultanément sur une même image.

5. Procédé selon la revendication 3, **caractérisé en ce que** la caméra (3) acquiert au moins deux images, une première image sur laquelle apparait au moins le repère facial (15) de l'individu (7), et une deuxième image sur laquelle apparait chaque marqueur (20,21) de la tablette (13), le traitement par calcul s'effectuant à partir des informations recueillies sur lesdites images.

6. Procédé selon la revendication 5 **caractérisé en ce que** la caméra (3) est déplacée entre une première position destinée à la capture de la première image et une deuxième position destinée à la capture de la deuxième image.

7. Procédé selon l'une quelconque des revendications précédentes, **caractérisé en ce que** le traitement de ladite au moins une image, comprend une étape de soustraction entre une premier vecteur reliant les yeux de l'individu (7) à l'appareil (3) d'acquisition de l'image, et un deuxième vecteur reliant la zone de lecture (28) et ledit appareil (3), et **en ce que** la restitution de l'information s'effectue par une lecture directe sur un écran (9).

8. Tablette (13) de lecture portable pour la mise en oeuvre d'un procédé selon l'une quelconque des revendications 1 à 7, ladite tablette comprenant une face (19) destinée à la lecture, et au moins un marqueur (20,21) de repérage spatial de ladite tablette (13), ledit au moins un marqueur (20,21) permettant un repérage même par faible luminosité, **caractérisée en ce qu'**elle comprend une première face (17,19) de lecture prolongée par une deuxième face (18) inclinée d'un angle de 10° à 80° par rapport à ladite première face (17), et **en ce que** ladite deuxième face (18) comprend trois marqueurs (20,21), l'un (20) en position frontale, et les deux autres (21) étant en position latérale, lesdits trois marqueurs (20,21) permettant de repérer la position dans l'espace de ladite tablette (13).

**Patentansprüche**

1. Verfahren zur Bestimmung des Leseabstands für eine Person (7), die veranlasst wird, einen Lesebereich (28) zu beobachten, das die folgenden Schritte enthält:

    - Positionieren der Person (7) vor einem Gerät (2), das konzipiert ist, mindestens ein Frontalbild der Person (7) zu erfassen, dann das Bild zu verarbeiten und schließlich eine Information wiederzugeben, die es der Person (7) ermöglicht, ihren Leseabstand zu kennen,
    - Lesen des Bereichs (28) durch die Person (7), der auf einem tragbaren Tablet (13) angeordnet und mit drei Ortungsmarkierern (20, 21) versehen ist, von denen einer (20) in frontaler Stellung auf dem Tablet (13) ist, die beiden anderen (21) in seitlicher Stellung sind, indem sie eine natürliche Haltung annimmt und die Stellung des Tablets (13) anpasst,
    - Erfassung durch das Gerät (2) mindestens eines Bilds, das es ermöglicht, mindestens einen Gesichtsbezugspunkt (15) der Person (7) und jeden Markierer (20, 21) des Lese-Tablets (13) anzuzeigen,
    - Verarbeitung durch Berechnungen des mindestens einen Bilds, unter Berücksichtigung der Stellung des Gesichtsbezugspunkts (15) der Person (7) und der Stellung der drei Markierer (20, 21) des Tablets (13),
    - Wiedergabe durch das Gerät (2) der den Leseabstand anzeigenden Information.

2. Verfahren nach Anspruch 1, **dadurch gekennzeichnet, dass** die Person (7) eine Brille (14) trägt, und dass der Gesichtsbezugspunkt durch Markierer (15) gebildet wird, die es unterschiedslos ermöglichen, entweder jedes der Augen (8) oder einen zentralen Punkt zwischen den Augen (8) zu markieren, und die fest mit einem Clip (16) verbunden sind, der an der Brille (14) befestigt ist.

3. Verfahren nach einem der vorhergehenden Ansprüche, **dadurch gekennzeichnet, dass** die angewendete Einrichtung, um mindestens ein Bild zu erfassen, eine Kamera (3) ist.

4. Verfahren nach Anspruch 3, **dadurch gekennzeichnet, dass** der Gesichtsbezugspunkt (15) der Person (7) sowie jeder Markierer (20, 21) des Tablets (13) gleichzeitig im gleichen Bild erscheinen.

5. Verfahren nach Anspruch 3, **dadurch gekennzeichnet, dass** die Kamera (3) mindestens zwei Bilder erfasst, ein erstes Bild, in dem mindestens der Gesichtsbezugspunkt (15) der Person (7) erscheint, und ein zweites Bild, in dem jeder Markierer (20, 21) des Tablets (13) erscheint, wobei die Verarbeitung durch Berechnung ausgehend von den in den Bildern gesammelten Informationen ausgeführt wird.

6. Verfahren nach Anspruch 5, **dadurch gekennzeichnet, dass** die Kamera (3) zwischen einer ersten Stellung, die zur Aufnahme des ersten Bilds bestimmt ist, und einer zweiten Stellung verschoben wird, die zur Aufnahme des zweiten Bilds bestimmt ist.

7. Verfahren nach einem der vorhergehenden Ansprüche, **dadurch gekennzeichnet, dass** die Verarbeitung des mindestens einen Bilds einen Schritt der Subtraktion zwischen einem ersten Vektor, der die Augen der Person (7) mit dem Aufnahmegerät (3) des Bilds verbindet, und einem zweiten Vektor enthält, der den Lesebereich (28) und das Gerät (3) verbindet, und dass die Wiedergabe der Information durch ein direktes Lesen auf einem Bildschirm (9) erfolgt.

8. Tragbares Lese-Tablet (13) zur Durchführung eines Verfahrens nach einem der Ansprüche 1 bis 7, wobei das Tablet eine zum Lesen bestimmte Seite (19) und mindestens einen Markierer (20, 21) zur räumlichen Markierung des Tablets (13) enthält, wobei der mindestens eine Markierer (20, 21) eine Markierung selbst bei geringer Helligkeit ermöglicht, **dadurch gekennzeichnet, dass** es eine erste Leseseite (17, 19) verlängert durch eine zweite Seite (18) enthält, die um einen Winkel von 10° bis 80° bezüglich der ersten Seite (17) geneigt ist, und dass die zweite Seite (18) drei Markierer (20, 21) enthält, einen (20) in frontaler Stellung und die zwei anderen (21) in seitlicher Stellung, wobei die drei Markierer (20, 21) es ermöglichen, die räumliche Stellung des Tablets (13) zu markieren.

**Claims**

1. Method for determining reading distance for an individual (7) made to observe a reading zone (28), comprising the following steps:

    - positioning the individual (7) in front of an apparatus (2) designed to acquire at least one front-on image of said individual (7), then to process said image and lastly to convey information allowing the individual (7) to learn their reading distance;
    - reading, by the individual (7), of said zone (28), which zone is placed on a portable tablet (13) and equipped with three location markers (20, 21), one (20) having a frontal position on said tablet (13), the two others (21) having lateral positions, the individual (7) adopting a natural position and adjusting the position of said tablet (13);
- acquiring, via the apparatus (2), at least one image allowing at least one facial reference point (15) of the individual (7) and each marker (20, 21) of the reading tablet (13) to be seen;
- calculationally processing said at least one image, taking into account the position of the facial reference point (15) of the individual (7) and the position of the three markers (20, 21) of said tablet (13); and
- conveying, via the apparatus (2), information indicating the reading distance.

2. Method according to Claim 1, **characterized in that** the individual (7) is wearing a pair of spectacles (14), and **in that** the facial reference point is marked by markers (15) that allow either each of the eyes (8), or a central point between said eyes (8), to be pinpointed, and that are securely fastened to a clip (16) fixed to said pair of spectacles (14).

3. Method according to either one of the preceding claims, **characterized in that** the means used to acquire the at least one image is a video camera (3).

4. Method according to Claim 3, **characterized in that** the facial reference point (15) of the individual (7) and each marker (20, 21) of the tablet (13) appears simultaneously in one and the same image.

5. Method according to Claim 3, **characterized in that** the video camera (3) acquires at least two images, a first image in which at least one facial reference point (15) of the individual (7) appears, and a second image in which each marker (20, 21) of the tablet (13) appears, the calculational processing being carried out on the basis of information gathered from said images.

6. Method according to Claim 5, **characterized in that** the video camera (3) is moved between a first position intended for the capture of the first image and a second position intended for the capture of the second image.

7. Method according to any one of the preceding claims, **characterized in that** the processing of said at least one image comprises a subtraction step between a first vector connecting the eyes of the individual (7) with the image acquiring camera (3), and a second vector connecting the reading zone (28) and said camera (3), and **in that** the information is conveyed by reading it directly from a display (9).

8. Portable reading tablet (13) for implementing a method according to any one of Claims 1 to 7, said tablet comprising a face (19) intended for reading, and at least one marker (20, 21) for pinpointing the spatial location of said tablet (13), said at least one marker (20, 21) allowing the tablet (13) to be located even under low-luminosity conditions, **characterized in that** it comprises a reading first face (17, 19) extended by a second face (18) inclined at an angle of 10° to 80° relative to said first face (17), and **in that** said second face (18) comprises three markers (20, 21), one (20) having a frontal position, and two others (21) having lateral positions, said three markers (20, 21) allowing the position of said tablet (13) to be pinpointed in space.

**Fig. 1**

**Fig. 2**

**Fig. 3a**

**Fig. 3b**

**Fig. 3c**

**EP 2 704 620 B1**

## RÉFÉRENCES CITÉES DANS LA DESCRIPTION

**Documents brevets cités dans la description**

- FR 2931002 A1

- US 20090262302 A